# EUROPÄISCHE PATENTSCHRIFT

(11) **EP 1 197 238 B1**
(45) Veröffentlichungstag und Bekanntmachung des Hinweises auf die Patenterteilung: **02.03.2005**
(21) Anmeldenummer: 01120289.2
(22) Anmeldetag: 24.08.2001
(51) Int. Cl.: A61M 16/20

(54) **Verfahren und Vorrichtung zur Veränderung eines Strömungsquerschnittes**
Method and device for changing the cross section of stream
Procédé et appareil pour modifier la section de passage d'un fluide

(30) Priorität: 12.10.2000 DE 10050443
(43) Veröffentlichungstag der Anmeldung: 17.04.2002
(73) Patentinhaber: Weinmann Geräte für Medizin GmbH & Co. KG, 22525 Hamburg (DE)
(72) Erfinder: Feldhahn, Karl-Andreas, Dr., 22761 Hamburg (DE); Ebers, Manfred, 25474 Bönningstedt (DE); Schulz, Gerd, 22869 Schenefeld (DE)
(74) Vertreter: Klickow, Hans-Henning, Dr.-Ing.

(56) Entgegenhaltungen:
- EP-A- 0 806 216
- EP-A- 0 990 448
- GB-A- 738 283
- US-A- 4 257 453
- US-A- 5 072 729
- US-A- 5 988 166
- US-A- 6 112 745

## Beschreibung

Die Erfindung betrifft ein Verfahren zur Veränderung eines Strömungsquerschnittes einer Luftableitung einer Beatmungseinrichtung, bei dem die Positionierung eines mindestens bereichsweise in die Luftableitung hineinragenden Drosselelementes verändert wird, sowie bei dem sowohl die Positionierung des Drosselelementes als auch der wirksame Strömungsquerschnitt in Abhängigkeit von einem Ausatmungsdruck verändert werden.

Die Erfindung betrifft darüber hinaus eine Vorrichtung zur Veränderung eines Strömungsquerschnittes im Bereich einer Luftableitung einer Beatmungseinrichtung, die ein Drosselelement umfaßt, das die Luftableitung mindestens bereichsweise vorgebbar versperrt, und bei der das Drosselelement mit einem Steuerelement zur Vorgabe eines wirksamen Strömungsquerschnittes verbunden ist, und bei der das Steuerelement eine Steuerkurve zur Vorgabe des wirksamen Strömungsquerschnittes in Abhängigkeit vom Ausatmungsdruck umfaßt und das Drosselelement in einer Positionierung zur teilweisen Versperrung der Luftableitung anordbar ist.

Mit Hilfe üblicher Beatmungseinrichtungen können Patienten mit Atemgas versorgt werden, wobei unterschiedliche Therapiedrücke vorgebbar sind. Grundsätzlich tritt das Problem auf, daß mit steigendem Therapiedruck auch die Luftmenge zunimmt, die über das jeweilige Ausatemsystem entweicht. Diese zunehmende Menge an Ausatemluft führt zu Geräuschbelästigungen beim Luftaustritt, darüber hinaus fühlt sich auch der Patient selbst durch die Menge der austretenden Luft belästigt und die erhöhte Strömung kann zu Unterkühlungen oder partiellen Hautaustrocknungen führen.

Aus der US-PS 60 06 784 (Resmed) ist es bereits bekannt, einen Volumenstrom von Atemgas in einem Ausströmungsbereich durch ein Ableitungsventil zu beeinflussen. Ein Positionierelement für das Drosselventil ist hierzu an einer Membran befestigt, die in Abhängigkeit vom jeweils einwirkenden Druck die ventilpositionierung vorgibt. Die Druckregulierung erfolgt somit in Form eines Bypasses, der über ein T-förmiges Kopplungselement an den Hauptströmungsweg anflanscht ist.

Aus der EP-OS 0 990 448 (Siemens-Elema) ist es bekannt, einen aufblasbaren Balg zu verwenden, um einen Strömungsquerschnitt in Abhängigkeit von einem Steuerdruck entweder völlig zu verschließen oder vollständig frei zu geben.

Aus der US-A-5,072,729 ist bereits eine Vorrichtung zur Veränderung eines Strömungsquerschnittes im Bereich einer Luftableitung einer Beatmungseinrichtung bekannt. Es wird hierbei ein Drosselelement verwendet, das die Luftableitung mindestens bereichsweise vorgebbar versperrt und das mit einem Steuerelement zur Vorgabe eines wirksamen Strömungsquerschnittes verbunden ist. An das Steuerelement ist ein Sensor zur Erfassung eines Ausatmungsdruckes im Bereich der Luftableitung angeschlossen. Das Drosselelement ist in einer Positionierung zur teilweisen Versperrung der Luftableitung anordbar. Die konstruktive Realisierung erfolgt im wesentlichen als ein Druckminderungsventil, das einen Ventilkörper als Absperrelement positioniert.

Aus der EP-A-0 806 216 ist eine Dosiereinrichtung zur Zuleitung von Narkosegasen zu einem Atemgas bekannt. Eine Dosierung erfolgt unter Verwendung eine Reihenschaltung von zwei Ventilen. Als Drosselelement kann ein Piezo-Element verwendet werden.

In der US-A-4,257,453 wird ein federbelastetes Ausatemventil für eine Beatmungseinrichtung beschrieben. Eine Absperrung des Strömungsweges erfolgt durch eine vorgebbare Positionierung eines Ventiltellers relativ zu einem Ventilsitz. Die Ventilsteuerung erfolgt mechanisch.

In der GB 738 283 wird ein weiteres Ventil beschrieben, das in Abhängigkeit von einer jeweiligen Ventilpositionierung vorgebbar einen Strömungsweg versperrt.

Aus der US-A-5,988,166 ist ein Ventil für eine Beatmungseinrichtung bekannt, bei dem eine ventilpositionierung über einen Schrittmotor vorgegeben wird. Als Drosselelement wird eine Blende eingesetzt.

Die bekannten Verfahren und Vorrichtungen sind nicht in ausreichender Weise dafür geeignet, im Bereich einer Beatmungseinrichtung eingesetzt zu werden und gleichzeitig einen möglichst gleichmäßigen Volumenstrom im Ausatmungsbereich als auch einen kompakten und funktionssicheren Aufbau zu gewährleisten.

Aufgabe der vorliegenden Erfindung ist es daher, ein Verfahren der einleitend genannten Art derart zu verbessern, daß bei einem kompakten Aufbau eine optimierte Luftableitung bereitgestellt wird.

Diese Aufgabe wird erfindungsgemäß dadurch gelöst, daß bei steigendem Ausatmungsdruck der Strömungsquerschnitt verkleinert und bei sinkendem Ausatmungsdruck vergrößert wird und daß der Ausatmungsdruck direkt auf das Drosselelement einwirkt.

Weitere Aufgabe der vorliegenden Erfindung ist es, eine Vorrichtung der einleitend genannten Art derart zu konstruieren, daß nachteilig hohe Volumenströme im Ausatmungsbereich bei kompaktem Aufbau vermieden werden.

Diese Aufgabe wird erfindungsgemäß dadurch gelöst, daß das Drosselelement eine Steuerung durch direkte Zuführung des Ausatmungsdruckes zum Drosselelement aufweist.

Ein besonders hoher Benutzungskomfort kann dadurch erreicht werden, daß die Veränderung des Strömungsquerschnittes derart erfolgt, daß ein annähernd konstanter Volumenfluß im Bereich der Luftableitung realisiert wird.

Zur Unterstützung einer einfachen Verfahrensdurchführung wird vorgeschlagen, daß eine Steuerung des Volumenflusses durchgeführt wird.

Eine besonders hohe Gleichmäßigkeit des Volumenflusses kann dadurch erreicht werden, daß eine Regelung des Volumenflusses unter Einbeziehung einer meßtechnischen Erfassung des Volumenflusses durchgeführt wird.

Eine gleichmäßige Ausströmung kann dadurch unterstützt werden, daß die Ausatmungsluft durch einen Umfangsspalt abgeleitet wird. Alternativ zu einem Umfangsspalt können beispielsweise auch mehrere separate Ausströmöffnungen oder amorphe beziehungsweise luftdurchlässige Ausströmmaterialien verwendet werden.

Insbesondere ist daran gedacht, daß die Ausatmungsluft durch einen im wesentlichen konzentrisch zu einer Stutzenlängsachse verlaufenden Ausströmspalt abgeleitet wird.

Ein Ausströmvorgang mit geringer Geräuschemission kann dadurch unterstützt werden, daß eine Veränderung des wirksamen Strömungsquerschnittes mindestens entlang eines überwiegenden Anteiles des Verlaufes des Ausströmspaltes durchgeführt wird.

Ein einfacher gerätetechnischer Aufbau wird auch dadurch unterstützt, daß der wirksame Strömungsquerschnitt durch die Wölbung einer Membran vorgegeben wird.

Bei einer Realisierung einer elektrischen Ansteuerbarkeit erweist es sich als vorteilhaft, daß der wirksame Strömungsquerschnitt durch die Durchbiegung eines Piezo-Elementes vorgegeben wird.

Zur Bereitstellung eines kompakten gerätetechnischen Aufbaues trägt es bei, daß das Drosselelement im Bereich eines Ausatmungselementes angeordnet ist, das einen Innenstutzen sowie einen Außenstutzen aufweist.

Bei der Verwendung von tubusförmigen Bauelementen erweist es sich als vorteilhaft, daß sich zwischen dem Innenstutzen und dem Außenstutzen ein Ausströmspalt erstreckt.

Eine zweckmäßige Materialauswahl besteht darin, daß das Drosselelement mindestens bereichsweise aus einem Elastomer ausgebildet ist.

Ein äußerst kompakter Aufbau unter vermeidung von Steuerleitungen und separaten Stellelementen kann dadurch erreicht werden, daß das Drosselelement eine Steuerung durch direkte Zuführung des Ausatmungsdruckes aufweist.

In der Zeichnung sind Ausführungsbeispiele der Erfindung schematisch dargestellt. Es zeigen:
- Fig. 1:: eine perspektivische vereinfachte Darstellung einer Beatmungseinrichtung, die aus einer Versorgungseinheit, einem Verbindungsschlauch sowie einer Beatmungsmaske ausgebildet ist,
- Fig. 2:: einen vergrößerten Querschnitt durch ein Ausatmungselement, das im Bereich des Verbindungsschlauches zwischen der Versorgungseinheit und der Beatmungsmaske anordbar ist
und
- Fig. 3:: ein vereinfachtes schematisches Blockschaltbild zur Veranschaulichung einer Regelung des Volumenstromes im Ausatmungsbereich.

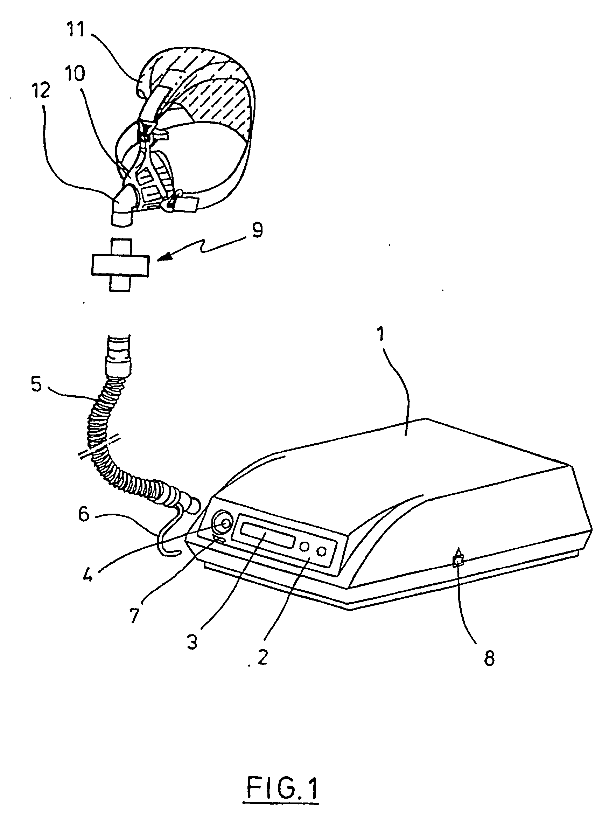

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) einen Anschlußstutzen (12) auf.

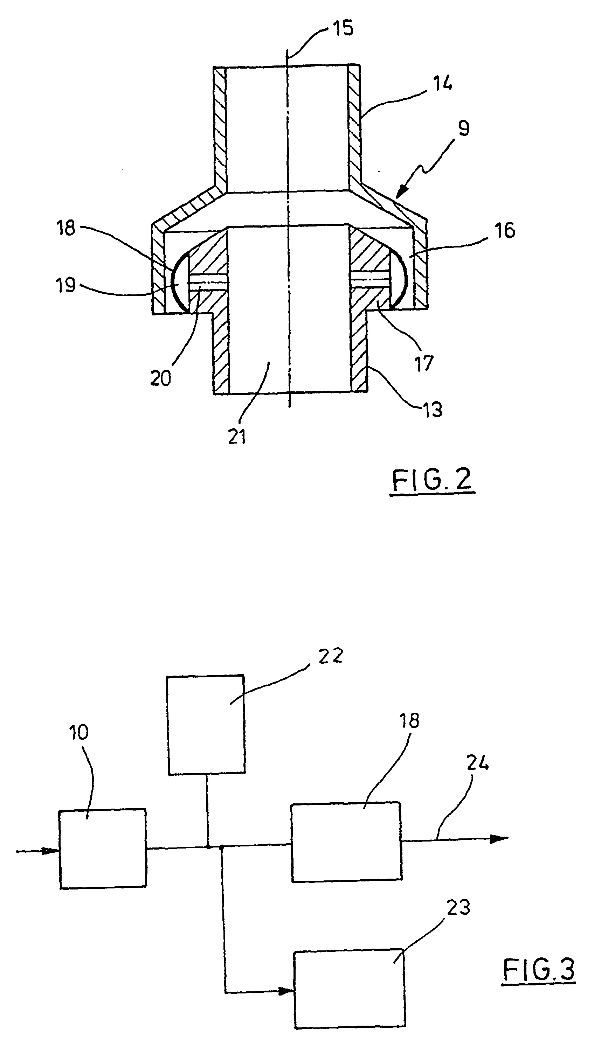

Fig. 2 zeigt einen Querschnitt durch das Ausatmungselement (9) in einer vergrößerten Darstellung. Es ist erkennbar, daß das Ausatmungselement (9) aus einem Innenstutzen (13) sowie einem Außenstutzen (14) ausgebildet ist. Der Außenstutzen (14) erstreckt sich in einem Teilbereich seiner Ausdehnung entlang einer Stutzenlängsachse (15) mit einem Abstand zum Innenstutzen (13) und begrenzt hierdurch gemeinsam mit dem Innenstutzen (13) einen Ausströmspalt (16).

Bei der dargestellten Ausführungsform weisen der Innenstutzen (13) und der Außenstutzen (14) eine im wesentlichen konzentrische Gestaltung bezüglich der Stutzenlängsachse (15) auf. Im Bereich seiner der Beatmungsmaske (10) zuwendbaren Ausdehnung besitzt der Außenstutzen (14) einen Außendurchmesser, der im wesentlichen dem Außendurchmesser des Innenstutzens (13) im Bereich dessen dem Verbindungsschlauch (5) zuwendbaren Ausdehnung entspricht. In Richtung auf den Innenstutzen (13) erweitert sich der Außenstutzen (14). Der Innenstutzen (13) ist im Bereich seiner dem Außenstutzen (14) zugewandten Ausdehnung ebenfalls mit einem sich erweiterndem Endsegment (17) ausgestattet, in dessen Bereich ein Drosselelement (18) angeordnet ist, daß in den Ausströmspalt (16) hineinragt, der die vorgesehene Luftableitung bereitstellt.

Bei der dargestellten Ausführungsform ist das Drosselelement (18) als eine wölbungsfähige Membran ausgebildet, die einen Steuerinnenraum (19) überspannt, der über einen Verbindungskanal (20) mit einem Hauptströmungsweg (21) verbunden ist, der durch den Innenstutzen (13) sowie den Außenstutzen hindurch vom verbindungsschlauch zur Beatmungsmaske (10) verläuft. Alternativ ist beispielsweise auch die Verwendung einer Rollmembran möglich.

Bei einem steigenden Druck im Bereich des Hauptströmungsweges (21) überträgt sich dieser Druck durch den Verbindungskanal (20) zum Steuerinnenraum (19). Der Steuerinnenraum (19) ist durch das membranförmige Drosselelement (18) gegenüber einem Umgebungsdruck beziehungsweise einem sich innerhalb des Endbereiches des Ausströmspaltes (16) einstellenden Druck abgeschlossen. Bei einem zunehmenden Druck im Bereich des Hauptströmungsweges (21) wird das Drosselelement (18) deshalb zunehmend in den Ausströmspalt (16) hineingedrückt und verengt diesen. Durch die Verengung des Ausströmspaltes (16) wird eine Verminderung des Volumenstromes des ausströmenden Atemgases erreicht.

Die in Fig. 2 dargestellte Ausführungsform vermeidet somit die Verwendung zusätzlicher Steuerungselemente. Ein Steuerelement für das Drosselelement (18) wird durch den Steuerinnenraum (19) bereitgestellt, der in Abhängigkeit von seinem Innendruck eine jeweilige Auswölbung des Drosselelementes (18) vorgibt. In diesem Fall wird der Druck im Bereich des Hauptströmungsweges (21) durch einen einfachen Verbindungskanal (20) direkt zum Steuerinnenraum (19) übertragen. Durch eine jeweilige Dimensionierung des Steuerinnenraumes (19), der vom Druck beaufschlagten Fläche des membranförmigen Drosselelementes (18) sowie der vorliegenden Membranelastizität wird eine Steuerkennlinie bereitgestellt, die eine jeweilige Querschnittverminderung des Ausströmspaltes (16) in Abhängigkeit vom Ausatmungsdruck definiert. Durch eine geeignete Abstimmung dieser Steuerungsparameter kann erreicht werden, daß eine möglichst flache Ausatmungskennlinie bereitgestellt wird, die unabhängig vom konkret vorliegenden Ausatmungsdruck einen möglichst konstanten Volumenfluß des abgeleiteten Atemgases gewährleistet.

Grundsätzlich ist es zwar denkbar, gehört aber nicht zur Erfindung, daß keine unmittelbare Ableitung des Steuerdruckes für das Drosselelement (18) durch eine Druckverbindung mit dem Hauptströmungsweg (21) erfolgt, sondern daß eine separate meßtechnische Erfassung über einen Drucksensor und eine separate Beaufschlagung mit einer Stellgröße erfolgt.

Statt eines membranförmigen Drosselelementes (18) können andersartige Drosselelemente (18) zur Veränderung des Strömungsquerschnittes des Ausströmspaltes (16) verwendt werden. Gedacht ist beispielsweise an den Einsatz von Piezoelementen, die in Abhängigkeit von einer Durchbiegung aufgrund einer angelegten elektrischen Steuerspannung eine jeweilige wirksame Querschnittfläche festlegen.

Gemäß einer weiteren Ausführungsform ist es auch möglich, das Drosselelement (18) im Bereich des Außenstutzens (14) zu haltern. Ebenfalls ist es möglich, das Drosselelement (18) als ein relativ sowohl zum Innenstutzen (13) als auch zum Außenstutzen (14) separates Bauteil zu realisieren.

Im Hinblick auf eine kompakte Ausführungsform erweist es sich als insbesondere als vorteilhaft, das Drosselelement (18) derart anzuordnen, daß bei einer Veränderung des wirksamen Strömungsquerschnittes eine Bewegungskomponente des Drosselelementes (18) in einer radialen Richtung relativ zur Stutzenlängsachse (15) vorliegt.

Bei einem Einsatz eines membranförmigen Drosselelementes (18) wird das Drosselelement (18) vorzugsweise aus einem elastomeren Werkstoff realisiert. Dies hat den Vorteil, daß das Drosselelement (18) aufgrund der auftretenden Eigenspannungen nach einem Nachlassen der Druckbeaufschlagung automatisch in die Ausgangsposition zurückkehrt.

Eine Festlegung der Steuerkennlinie des Drosselelementes (18) erfolgt derart, daß die aus dem Ausströmspalt (16) austretende Luftmenge gerade so groß gewählt wird, daß das im Bereich der Beatmungsmaske (10) enthaltene Kohlendioxid sicher ausgewaschen wird und daß andererseits die austretende Luftmenge klein genug gewählt wird, um nachteilige Wirkungen zu vermeiden.

Fig. 3 zeigt ein schematisches Blockschaltbild zur weiteren Erläuterung des Prinzips bei der Realisierung einer Regelung. Es ist erkennbar, daß im Verbindungsbereich zwischen der Beatmungsmaske (10) und einer Druckversorgung (22) im Bereich eines Ausströmweges (24) das Drosselelement (18) angeordnet ist, dessen Positionierung von einer Steuereinheit (23) vorgegeben wird. Der Steuereinheit kann in einer gerätetechnischen einfachen Ausführungsform der Ausatmungsdruck als Eingangsgröße zugeführt werden. Generell ist es auch denkbar, meßtechnisch den Volumenfluß zu ermitteln und eine direkte Regelung des Volumenflusses durch geeignete Einstellungen des Drosselelementes (18) vorzunehmen.

Alternativ zu den bereits erläuterten Ausbildungsmöglichkeiten für das Drosselelement (18) ist es beispielsweise auch möglich, das Drosselelement (18) aus zwei relativ zueinander beweglichen Elementen zu realisieren. Hierdurch wird der erforderliche Hub je Element vermindert. Beispielsweise ist es möglich, zwei relativ zueinander gegenüberliegende Membranen einzusetzen. Eine andere Ausführungsform besteht darin, das Drosselelement (18) als eine Blende auszubilden, die mit einer hinsichtlich ihrer Querschnittfläche veränderlichen Durchtrittsöffnung realisiert. Eine derartige Blende kann beispielsweise als ein expandierbarer Ringbalg realisiert sein.

## Patentansprüche

1. Verfahren zur Veränderung eines Strömungsquerschnittes einer Luftableitung einer Beatmungseinrichtung, bei dem die Positionierung eines mindestens bereichsweise in die Luftableitung hineinragenden Drosselelementes (18) verändert wird, sowie bei dem sowohl die Positionierung des Drosselelementes (18) als auch der wirksame Strömungsquerschnitt in Abhängigkeit von einem Ausatmungsdruck verändert wird, **dadurch gekennzeichnet, daß** bei steigendem Ausatmungsdruck der Strömungsquerschnitt verkleinert und bei sinkendem Ausatmungsdruck vergrößert wird und daß der Ausatmungsdruck direkt auf das Drosselelement einwirkt.

2. Verfahren nach Anspruch 1, **dadurch gekennzeichnet, daß** die Veränderung des Strömungsquerschnittes derart erfolgt, daß ein annähernd konstanter Volumenfluß im Bereich der Luftableitung realisiert wird.

3. Verfahren nach Anspruch 1 oder 2, **dadurch gekennzeichnet, daß** eine Steuerung des Volumenflusses durchgeführt wird.

4. Verfahren nach Anspruch 1 oder 2, **dadurch gekennzeichnet, daß** eine Regelung des Volumenflusses unter Einbeziehung einer meßtechnischen Erfassung des Volumenflusses durchgeführt wird.

5. Verfahren nach einem der Ansprüche 1 bis 4, **dadurch gekennzeichnet, daß** die Ausatmungsluft durch einen Umfangsspalt abgeleitet wird.

6. Verfahren nach Anspruch 5, **dadurch gekennzeichnet, daß** die Ausatmungsluft durch einen im wesentlichen konzentrisch zu einer Stutzenlängsachse (15) verlaufenden Ausströmspalt (16) abgeleitet wird.

7. Verfahren nach einem der Ansprüche 1 bis 6, **dadurch gekennzeichnet, daß** eine Veränderung des wirksamen Strömungsquerschnittes mindestens entlang eines überwiegenden Anteiles des Verlaufes des Ausströmspaltes (16) durchgeführt wird.

8. Verfahren nach einem der Ansprüche 1 bis 7, **dadurch gekennzeichnet, daß** der wirksame Strömungsquerschnitt durch die Wölbung einer Membran vorgegeben wird.

9. Verfahren nach einem der Ansprüche 1 bis 7, **dadurch gekennzeichnet, daß** der wirksame Strömungsquerschnitt durch die Durchbiegung eines Piezo-Elementes vorgegeben wird.

10. Vorrichtung zur Veränderung eines Strömungsquerschnittes im Bereich einer Luftableitung einer Beatmungseinrichtung, die ein Drosselelement (18) umfaßt, das die Luftableitung mindestens bereichsweise vorgebbar versperrt, und bei der das Drosselelement (18) mit einem Steuerelement zur Vorgabe eines wirksamen Strömungsquerschnittes verbunden ist, und bei der das Steuerelement eine Steuerkurve zur Vorgabe des wirksamen Strömungsquerschnittes in Abhängigkeit vom Ausatmungsdruck umfaßt und das Drosselelement (18) in einer Positionierung zur teilweisen Versperrung der Luftableitung anordbar ist, **dadurch gekennzeichnet, daß** das Drosselelement (18) eine Steuerung durch direkte Zuführung des Ausatmungsdruckes zum Drosselelement (18) aufweist.

11. Vorrichtung nach Anspruch 10, **dadurch gekennzeichnet, daß** das Drosselelement (18) im Bereich eines Ausatmungselementes (9) angeordnet ist, das einen Innenstutzen (13) sowie einen Außenstutzen (14) aufweist.

12. Vorrichtung nach Ansprüche 10 oder 11, **dadurch gekennzeichnet, daß** sich zwischen dem Innenstutzen (13) und dem Außenstutzen (14) ein Ausströmspalt (16) erstreckt.

13. Vorrichtung nach Anspruch 12, **dadurch gekennzeichnet, daß** der Ausströmspalt (16) relativ zu einer Stutzenlängsachse (15) im wesentlichen kreisförmig verläuft.

14. Vorrichtung nach einem der Ansprüche 10 bis 13, **dadurch gekennzeichnet, daß** das Drosselelement (18) membranartig ausgebildet ist.

15. Vorrichtung nach einem der Ansprüche 10 bis 13, **dadurch gekennzeichnet, daß** das Drosselelement (18) mindestens bereichsweise als ein Piezo-Element ausgebildet ist.

16. Vorrichtung nach einem der Ansprüche 10 bis 15, **dadurch gekennzeichnet, daß** das Drosselelement (18) an eine mechanische Steuerung für den Volumenfluß angeschlossen ist.

17. Vorrichtung nach einem der Ansprüche 10 bis 15, **dadurch gekennzeichnet, daß** das Drosselelement (18) an eine Regelung für den Volumenfluß angeschlossen ist.

18. Vorrichtung nach einem der Ansprüche 10 bis 17, **dadurch gekennzeichnet, daß** das Drosselelement (18) mindestens bereichsweise aus einem Elastomer ausgebildet ist.

19. Vorrichtung nach einem der Ansprüche 10 bis 18, **dadurch gekennzeichnet, daß** das Drosselelement (18) mindestens zwei relativ zueinander positionierbare Elemente aufweist.

20. Vorrichtung nach einem der Ansprüche 10 bis 18, **dadurch gekennzeichnet, daß** das Drosselelement (18) als eine Blende mit veränderlicher Austrittsfläche ausgebildet ist.

## Claims

1. Method of varying a flow cross-section of an air discharge line of a ventilator, whereby the positioning of a throttle element (18) extending into at least certain regions of the air discharge line is varied, and whereby both the positioning of the throttle element (18) and the active flow cross-section are varied depending on an exhalation pressure, **characterised in that** the flow cross-section is reduced as the exhalation pressure increases and increased as the exhalation pressure decreases and the exhalation pressure acts directly on the throttle element.

2. Method as claimed in claim 1, **characterised in that** the flow cross-section is varied in such a way that a virtually constant volumetric flow is obtained in the region of the air discharge line.

3. Method as claimed in claim 1 or 2, **characterised in that** the volumetric flow is controlled.

4. Method as claimed in claim 1 or 2, **characterised in that** the volumetric flow is automatically controlled on the basis of a sensed measurement of the volumetric flow.

5. Method as claimed in one of claims 1 to 4, **characterised in that** the exhaled air is discharged through a circumferential gap.

6. Method as claimed in claim 5, **characterised in that** the exhaled air is discharged through an outlet gap (16) extending essentially concentrically with a connector piece longitudinal axis (15).

7. Method as claimed in one of claims 1 to 6, **characterised in that** the effective flow cross-section is varied at least along a predominant proportion of the length of the outlet gap (16).

8. Method as claimed in one of claims 1 to 7, **characterised in that** the effective flow-cross section is determined by the camber of a membrane.

9. Method as claimed in one of claims 1 to 7, **characterised in that** the effective flow cross-section is determined by the bending of a piezo-element.

10. Device for varying a flow-cross section of a respirator in the region of an air discharge line, comprising a throttle element (18) which shuts off at least certain regions of the air discharge line by a pre-settable amount, in which the throttle element (18) is connected to a control element for pre-setting an effective flow cross-section, and in which the control element uses a control curve for pre-setting the effective flow cross-section depending on the exhalation pressure, and the throttle element (18) can be disposed in a specific positioning so as to partially shut off the air discharge line, **characterised in that** the throttle element (18) has a control system which transmits the exhalation pressure directly to the throttle element (18).

11. Device as claimed in claim 10, **characterised in that** the throttle element (18) is disposed in the region of an exhalation element (9) which has an inner connector (13) piece and an outer connector piece (14).

12. Device as claimed in claims 10 or 11, **characterised in that** an outlet gap (16) extends between the inner connector piece (13) and the outer connector piece (14).

13. Device as claimed in claim 12, **characterised in that** the outlet gap (16) extends in a substantially annular arrangement relative to a connector piece longitudinal axis (15).

14. Device as claimed in one of claims 10 to 13, **characterised in that** the throttle element (18) is of a membrane-type design.

15. Device as claimed in one of claims 10 to 13, **characterised in that** the throttle element (18) is provided in the form of a piezo-element, at least in certain regions.

16. Device as claimed in one of claims 10 to 15, **characterised in that** the throttle element (18) is connected to a mechanical control for the volumetric flow.

17. Device as claimed in one of claims 10 to 15, **characterised in that** the throttle element (18) is connected to an automatic control for the volumetric flow.

18. Device as claimed in one of claims 10 to 17, **characterised in that** the throttle element (18) is provided in the form of an elastomer, at least in certain regions.

19. Device as claimed in one of claims 10 to 18, **characterised in that** the throttle element (18) has at least two elements which can be positioned relative to one another.

20. Device as claimed in one of claims 10 to 18, **characterised in that** the throttle element (18) is provided in the form of a diaphragm with a variable outlet surface.

## Revendications

1. Procédé pour la modification d'une section de passage d'une conduite d'évacuation d'air d'un respirateur dans le cadre duquel le positionnement d'un organe d'étranglement (18), qui pénètre, au moins en partie, dans la conduite d'évacuation, est modifié et dans le cadre duquel aussi bien le positionnement de l'élément d'étranglement (18) que la section transversale effective d'écoulement sont modifiés en dépendance d'une pression d'expiration, **caractérisé en ce que** la section transversale d'écoulement est réduite quand la pression d'expiration augmente, et est agrandie quand la pression d'expiration diminue, et **en ce que** la pression d'expiration agit directement sur l'élément d'étranglement.

2. Procédé selon la revendication 1, **caractérisé en ce que** la modification de la section transversale d'écoulement est effectuée de sorte qu'un débit volumétrique à peu près constant est obtenu dans la zone de la conduite d'évacuation d'air

3. Procédé selon la revendication 1 ou 2, **caractérisé en ce qu'**une commande du débit volumétrique est exécutée.

4. Procédé selon la revendication 1 ou 2, **caractérisé en ce qu'**une régulation du débit volumétrique est effectuée, incluant une détermination du débit volumétrique par une technique de mesure.

5. Procédé selon la revendication 1 à 4, **caractérisé en ce que** l'air de respiration est évacué par une fente périphérique.

6. Procédé selon la revendication 5, **caractérisé en ce que** l'air de respiration est évacué par une fente d'écoulement (16) sensiblement orientée concentriquement par rapport à un axe longitudinal (15) d'une tubulure.

7. Procédé selon une revendication 1 à 6, **caractérisé en ce qu'**une modification de la section transversale effective est effectuée au moins le long d'une majeure partie de l'étendue de la fente d'écoulement (16).

8. Procédé selon une revendication 1 à 7, **caractérisé en ce que** la section transversale effective est prédéterminée par la courbure d'une membrane.

9. Procédé selon une revendication 1 à 7, **caractérisé en ce que** la section transversale effective est prédéterminée par la flexion d'un élément piézoélectrique.

10. Dispositif pour la modification d'une section transversale d'écoulement d'une sortie d'air d'un respirateur qui comprend un élément d'étranglement (18) qui obstrue de manière prédéterminée au moins partiellement la conduite d'évacuation d'air et dans lequel l'élément d'étranglement (18) est relié à un élément de commande pour la prédétermination d'une section transversale d'écoulement effective, et dans lequel l'élément de commande comprend une courbe de commande pour la prédétermination de la section transversale d'écoulement effective en dépendance de la pression d'expiration, et l'élément d'étranglement (18) peut être disposé dans une position pour l'obturation partielle de la conduite d'évacuation d'air, **caractérisé en ce qu'**au niveau de l'élément d'étranglement (18) une commande est prévue pour l'arrivée directe de la pression d'expiration audit élément d'étranglement (18).

11. Dispositif selon la revendication 10, **caractérisé en ce que** l'élément d'étranglement (18) est disposé dans la zone d'un respirateur (9) qui présente une tubulure intérieure (13) ainsi qu'une tubulure extérieure (14).

12. Dispositif selon la revendication 10, **caractérisé en ce qu'**entre la tubulure intérieure (13) et la tubulure extérieure (14), s'étend une fente d'écoulement (16).

13. Dispositif selon la revendication 10, **caractérisé en ce que** la fente d'écoulement (16) s'étend sensiblement circulairement par rapport à un axe longitudinal (15) des tubulures.

14. Dispositif selon une revendication 10 à 13, **caractérisé en ce que** l'élément d'étranglement (18) est conçu dans le genre d'une membrane.

15. Dispositif selon l'une des revendications 10 à 13, **caractérisé en ce que** l'élément d'étranglement (18) est conçu au moins en partie comme un organe piézoélectrique.

16. Dispositif selon une revendication 10 à 15, **caractérisé en ce que** l'élément d'étranglement (18) est raccordé à une commande mécanique du débit volumétrique.

17. Dispositif selon une revendication 10 à 15, **caractérisé en ce que** l'élément d'étranglement (18) est raccordé à un système de régulation du débit volumétrique.

18. Dispositif selon une revendication 10 à 17, **caractérisé en ce que** l'élément d'étranglement (18) est réalisé au moins en partie en élastomère.

19. Dispositif selon une revendication 10 à 18, **caractérisé en ce que** l'élément d'étranglement (18) présente au moins deux éléments qui peuvent être positionnés l'un par rapport à l'autre.

20. Dispositif selon une revendication 10 à 18, **caractérisé en ce que** l'élément d'étranglement (18) est conçu sous la forme d'un diaphragme à ouverture variable.
